# EUROPEAN PATENT SPECIFICATION

(11) **EP 3 982 941 B1**
(45) Date of publication and mention of the grant of the patent: **19.08.2026**
(21) Application number: 20743259.2
(22) Date of filing: 15.06.2020
(51) Int. Cl.: A61K 9/51, A61B 5/00, A61K 31/00, A61K 49/00, A61K 51/00, B01J 13/00

(54) **METHOD FOR THE PREPARATION OF LIPID NANOPARTICLES**
VERFAHREN ZUR HERSTELLUNG VON LIPIDNANOPARTIKELN
PROCÉDÉ DE PRÉPARATION DE NANOPARTICULES LIPIDIQUES

(30) Priority: 17.06.2019 IT 201900009258
(43) Date of publication of application: 20.04.2022
(73) Proprietor: R Bio Transfer S.r.l., 84131 Salerno (SA) (IT)
(72) Inventor: BATTAGLIA, Luigi Sebastiano, 84131 Salerno (SA) (IT); DIANZANI, Chiara, 84131 Salerno (SA) (IT); MUNTONI, Elisabetta, 84131 Salerno (SA) (IT); SCORZIELLO, Franco, 84131 Salerno (SA) (IT)
(74) Representative: Brunacci, Marco
(86) International application number: PCT/IB2020/055562
(87) International publication number: WO 2020/254934

(56) References cited:
- WO-A1-2018/109690
- SHAH ROHAN M ET AL: "Physicochemical characterization of solid lipid nanoparticles (SLNs) prepared by a novel microemulsion technique", JOURNAL OF COLLOID AND INTERFACE SCIENCE, ACADEMIC PRESS,INC, US, vol. 428, 4 May 2014 (2014-05-04), pages 286 - 294, XP028850061, ISSN: 0021-9797, DOI: 10.1016/J.JCIS.2014.04.057

## Description

### Technical Field

The present invention relates to a method for the preparation of lipid nanoparticles.

### Background Art

The use of lipid nanoparticles in the pharmaceutical field for conveying pharmaceutical active ingredients is well known and widespread.

With particular reference to the pharmaceutical field, numerous methods for the preparation of lipid nanoparticles are known.

One of the most widely used techniques for the production of lipid nanoparticles is that of high pressure homogenization, which can be carried out either hot or cold. In both cases the drug is dissolved or solubilized in the melted lipid matrix (5-10°C above melting temperature). In the case of the hot homogenization technique, the resulting mixture is dispersed under agitation in an aqueous surfactant solution, previously brought to the same temperature as the lipids. A pre-emulsion is thus obtained which is homogenized by means of high-pressure homogenizers and then cooled to crystallize the lipids and obtain solid lipid nanoparticles.

However, the hot technique cannot be used to incorporate into the nanoparticles thermolabile drugs, which would be degraded due to the high temperatures, or hydrophilic drugs, which could break down in the aqueous phase during homogenization.

This method has been described in patent document EP0605497 and has many drawbacks, including the fact that it requires the use of complex and expensive instruments, as well as the use of high temperatures.

In addition to this, the high cavitation forces generated determine the alteration of the conveyed active ingredient.

An alternative preparation method is based on the formation of hot micro-emulsions as precursors of the lipid nanoparticles.

Micro-emulsions are stable and transparent biphasic mixtures consisting of two immiscible liquids, water and oil, stabilized by a surfactant and a co-surfactant.

In particular, the size of the internal phase makes them suitable as precursors of nanoparticle systems.

This method, described in the patent document US1993/5250236, consists in the dilution of the micro-emulsion in cold water in order to break it and cause the precipitation of the particles formed by the solidified lipids. However, even this method is not without its drawbacks.

In fact, one of the disadvantages of this method is related to the dilution of the initial micro-emulsion which results in obtaining a diluted suspension of nanoparticles, to which must be added the fact that this method is complex to perform on a large scale.

In order to overcome at least part of these drawbacks, a method has been developed for the preparation of lipid nanoparticles, described in the patent document US2006/0292183, which makes it possible to obtain lipid nanoparticles by simple cooling of an oil micro-emulsion in water.

However, this method makes it possible to obtain a lipid concentration that can be used for the realization of an extremely low 0.2% w/v hot stable micro-emulsion and a cold nanoparticle suspension, greatly reducing the possibility of loading the active ingredient.

A further method of preparing lipid nanoparticles, described in patent document WO 01/64328, consists in heating and cooling a mixture. This method is based on the principle of phase reversal, which requires the use of surfactants not approved by the Food and Drug Administration (FDA) and which therefore prevents their parenteral administration.

WO 2018/109690 A1 discloses a one-pot process for the formation of lipid nanoparticles, whereby the lipid is chosen from a mixture of solid and liquid triglycerides, a non-ionic surfactant and, optionally, a co-surfactant. The components are heated to below 100 °C and the resulting micro-emulsions are cooled to room-temperature.

### Description of the Invention

The main aim of the present invention is to provide a method for the preparation of lipid nanoparticles which makes it possible to prepare solid nanoparticles in concentrated aqueous solution stabilized by biocompatible surfactants and co-surfactants.

A further object of the present invention is to devise a method for the preparation of lipid nanoparticles that allows considerably simplifying the preparation operations compared to methods of known type.

Another object of the present invention is to devise a method for the preparation of lipid nanoparticles that allows overcoming the above mentioned drawbacks of the prior art in a simple, rational, easy, effective to use and cost-effective solution.

### Brief Description of the Drawings

Other characteristics and advantages of the present invention will be more evident from the description of a preferred, but not exclusive, embodiment of a method for the preparation of lipid nanoparticles, illustrated by way of an indicative, yet non-limiting example, in the attached tables of drawings wherein:
Figure 1 is a graph drawn as a function of the thermal analysis of trimyristin nanoparticles produced with the method according to the present invention;
Figure 2 shows the comparison graph between the fractograms with UV detection of trimyristin 1, trimyristin 2 and trimyristin 3 produced by the method according to the invention;
Figures 3-5 are representative graphs of the FFF DLS fractogram of the nanoparticles of trimyristin 1, trimyristin 2 and trimyristin 3, respectively;
Figures 6 and 7 are column graphs representative of the comparative analysis carried out with regard to the efficacy of incorporation and loading of the dye of Trimyristin 1, Trimyristin 2 and Trimyristin 3, respectively, as a result of separation by gel filtration and separation by dextran centrifugation;
Figure 8 is a column chart of the cytotoxicity of trimyristin 2 lipid nanoparticles;
Figure 9-12 are column graphs of the comparative study on the biodistribution of lipid nanoparticles marked with 6-coumarin in Wistar rats after intravenous administration.

### Embodiments of the Invention

The present invention relates to a method for the preparation of solid lipid nanoparticles.

According to the invention, the method involves the phase of mixing in a single container a mixture comprising an aqueous solution with a solid lipid matrix and with at least one biocompatible non-ionic surfactant.

Hereunder, the term "lipid matrix" relates to a matrix of lipophilic nature, insoluble in water and soluble in organic solvents, with low melting point and comprising at least one of either triglyceride, a mixture of aliphatic or sterol esters.

Preferably, the aforementioned mixture comprises hydrocarbons. Advantageously, the surfactant is a polysorbate.

In detail, the surfactant is selected from the list comprising: Tween 20, Tween 40, Tween 60, Tween 80.

As an alternative, the surfactant is a derivative of sorbitan ethoxylate.

According to the invention, the mixture comprises a co-surfactant comprising an ester of sorbitan.

The co-surfactant is selected from the list comprising: Span 40, Span 60, Span 80.

The method comprises:
- the phase of heating the container to a working temperature of less than 100°C to obtain an emulsion;
- the phase of cooling the emulsion up to a temperature of less than 30°C to obtain solid lipid nanoparticles of a size of less than 500 nm.

The lipid matrix is present in a weight/volume concentration, evaluated with respect to the total weight of the aqueous solution, ranging from 0.3% to 5% and preferably from 0.5% to 4%.

Advantageously, the surfactant is present in a weight/volume concentration, evaluated with respect to the total weight of the aqueous solution, ranging from 1% to 20% and preferably from 2% to 16%.

Preferably, the co-surfactant is present in a weight/volume concentration, evaluated with respect to the total weight of the aqueous solution, ranging from 0.5% to 10%, preferably from 1% to 8%.

It should be pointed out that during the heating phase, the mixture is heated to a temperature higher than the lipid melting point and the cloud point of the surfactant, i.e. of the Tween, but lower than the boiling point of the water.

Under these conditions the Tween becomes insoluble in water, separates from the water solution of the same and interacts intimately with the surfactant, i.e. the Span, and with a melted lipid. This interaction takes place within a turbid-looking system in which the non-aqueous components of the mixture mix with each other.

Then, the method comprises a phase of purification of the solid lipid nanoparticles.

The phase of purification is selected from the group comprising: molecular exclusion chromatography, sedimentation and resuspension.

According to the invention, the solid lipid nanoparticles have a size ranging from 40 nm to 500 nm.

The present invention also relates to the use of solid lipid nanoparticles to formulate radio-frequency drugs, anticancer drugs, radiopharmaceuticals, and in sensors.

In particular, these anticancer drugs are drugs for organs with a mass. Examples of formulations of solid lipid nanoparticles are listed below.

### EXAMPLE 1

### Formulation of solid lipid nanoparticles of trimyristin and cholesteryl oleate.

The following table (Table 1) lists different formulations of solid lipid nanoparticles obtained by means of the method according to the present invention and comprising a lipid selected from trimyristin, cholesteryl palmitate or tripalmitin.

**Table 1**

| LIPID NANOPARTICLES | Lipid (mg) | Span 80 (µl) | Tween 20 (µl) | De-ionized water (ml) |
|---|---|---|---|---|
| Trimyristin 1 | 100 | 200 | 400 | 5 |
| Trimyristin 2 | 100 | 200 | 300 | 5 |
| Trimyristin 3 | 100 | 200 | 250 | 5 |
| Cholesteryl palmitate 1 | 100 | 200 | 400 | 5 |
| Cholesteryl palmitate 2 | 100 | 200 | 300 | 5 |
| Tripalmitin | 100 | 200 | 300 | 5 |

The lipid selected from Trimyristin and Cholesteryl palmitate was mixed with the aqueous solution, Tween 20 and Span 80. This solution was heated to 80°C, i.e. a temperature above the cloud point of Tween 20.

Subsequently this solution was cooled to 60°C, reaching this temperature permits the formation of a micro-emulsion.

It is specified that, in this treatise, the term "micro-emulsion" relates to a biphasic stable and transparent colored mixture, consisting of two immiscible liquids (water and oil) stabilized by a surfactant and generally by a co-surfactant.

In detail, the micro-emulsion is clear and the lipid nanoparticles are suspended therein.

Afterwards, the micro-emulsion is cooled down to room temperature, resulting in the precipitation of nanoparticles.

### EXAMPLE 2

### Thermal analysis of the trimyristin 2 nanoparticles

The thermal analysis was conducted by differential scanning calorimetry (DSC) on the nanoparticles in suspension both before and after the purification phase. In detail, the purification phase was performed by molecular exclusion (gel filtration technique with exclusion limit of 100000 Da).

In particular, the gel filtration removes the excess Tween 20, free and in micelles.

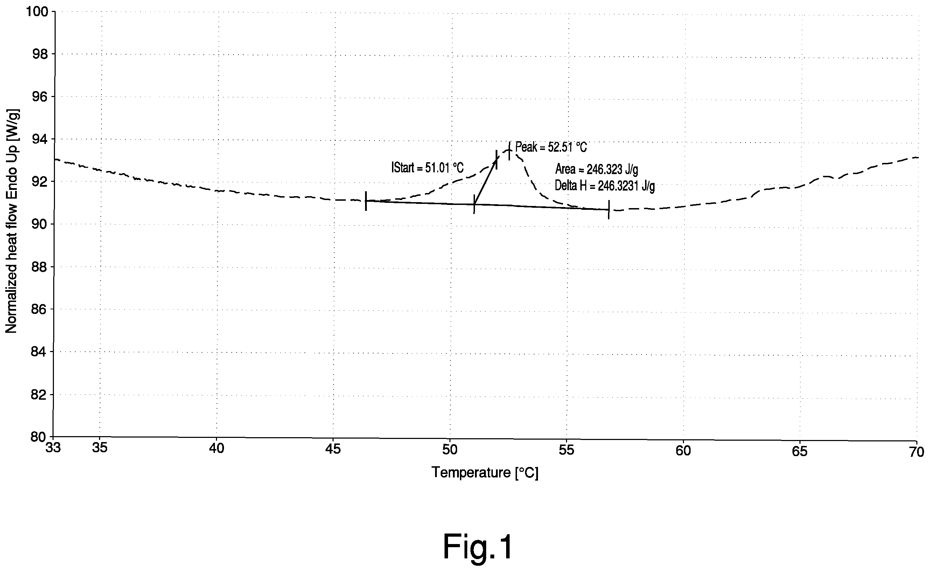

As can be seen in Figure 1, before purification, the micro-emulsion shows no phase transitions in light of the fact that the lipid melting temperature falls within the temperature range of existence and stability of the micro-emulsion.

Instead, the sample purified from micelles shows a melting transition typical of trimyristin.

### EXAMPLE 3

### Dimensional analysis of the trimyristin nanoparticles

The dimensional analysis of the trimyristin nanoparticles (Trimyristin 1, Trimyristin 2, Trimyristin 3) was conducted by means of Dynamic Light Scattering (DLS).

The table below (Table 2) shows the results obtained:

**Table 2**

| Lipid nanoparticles | Average diameter (nm) | Polydispersion |
|---|---|---|
| Trimyristin 1 | 158.1 | 0.140 |
| Trimyristin 2 | 251.4 | 0.019 |
| Trimyristin 3 | 424.3 | 0.157 |

### Dimensional analysis was further detailed with Field Flow Fractionation (FFF) with UV and DLS detection.

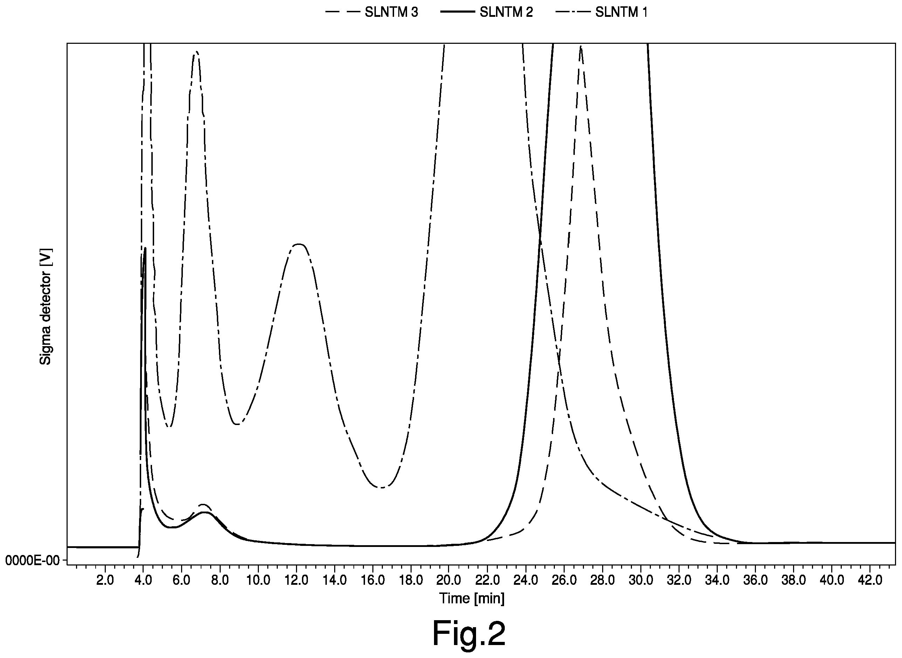

Figure 2 shows the comparison graph between the fractograms with UV detection of trimyristin 1, trimyristin 2 and trimyristin 3.

The peaks corresponding to Trimyristin 1, Trimyristin 2 and Trimyristin 3 were further analyzed with DLS detection.

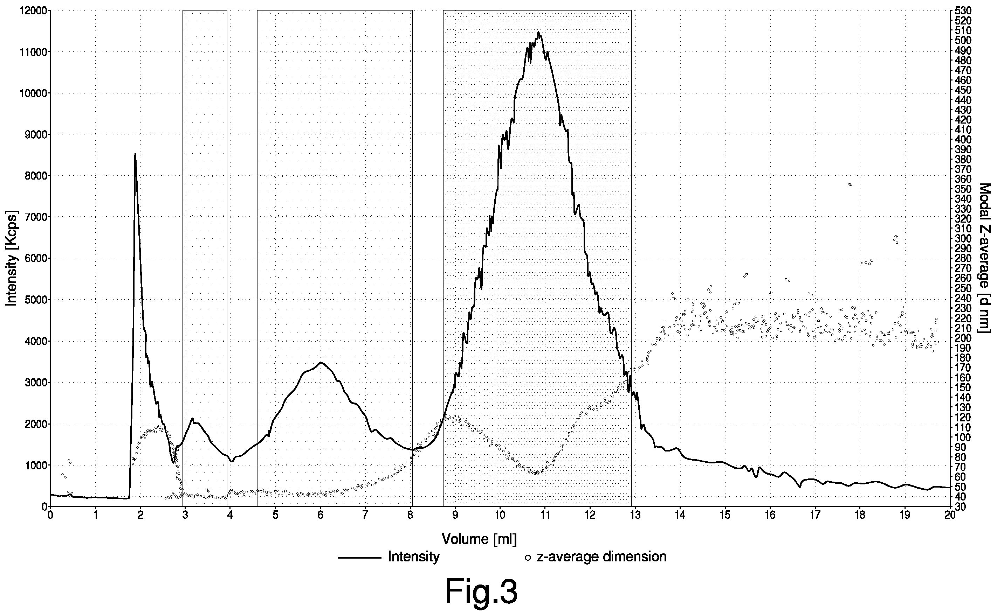

The Figures 3, 4 and 5 show the comparative graph of the fractograms obtained with FFF UV.

In particular, Figure 3 shows the FFF DLS fractogram of the nanoparticles of trimyristin 1 characterized by:
- peak 1: 38 nm;
- peak 2: 70 nm;
- peak 3: 136 nm.

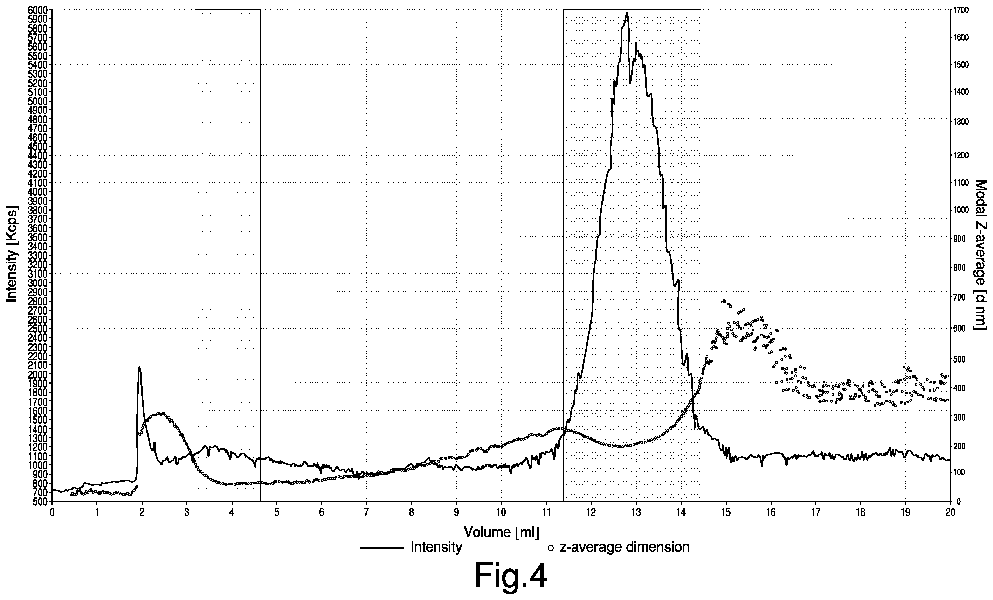

Likewise, Figure 4 shows the FFF DLS fractogram of the nanoparticles of trimyristin 2 characterized by:
- peak 1: 59 nm;
- peak 2: 202 nm.

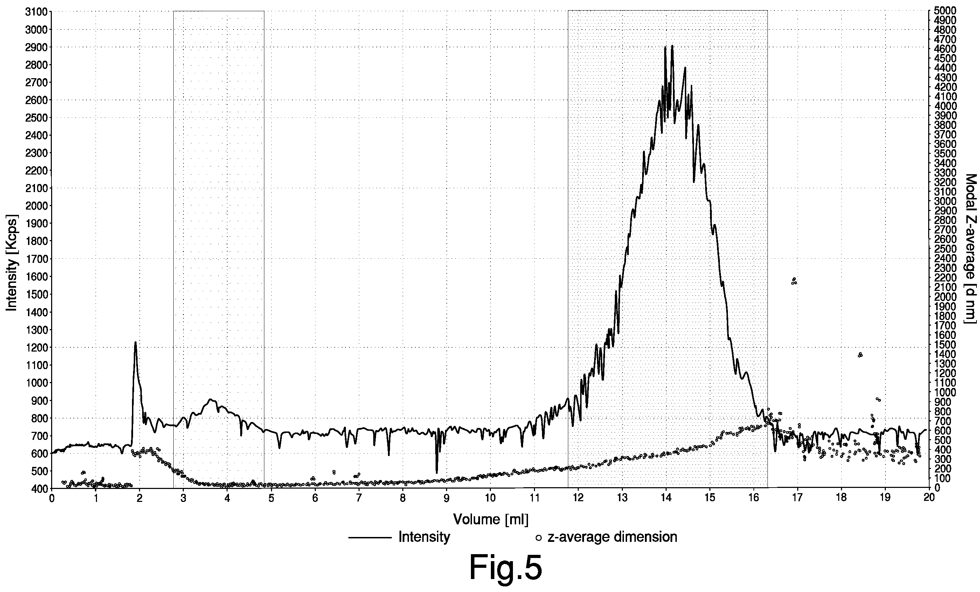

Again, Figure 5 shows the FFF DLS fractogram of the nanoparticles of trimyristin 3 characterized by:
- peak 1: 72 nm;
- peak 2: 407 nm.

The analysis of these graphs shows that the trimyristin 1 nanoparticles, which have a higher polydispersion than the DLS batch reading, are composed of a mixture of three populations. Those of trimyristin 2 and 3, on the other hand, are made up of a majority population, with a limited quantity of smaller nanoparticles.

The average size of the trimyristin 1 nanoparticles was further analyzed by DLS, after being purified with molecular exclusion techniques (gel filtration and gel centrifugation) and then sedimentation (centrifugation after 1:1 dilution with 30% dextran, followed by resuspension of the precipitate in water). The gel filtration has an exclusion limit of 100000 Da; this means that this type of technique excludes the Tween 20 in monomer form and in micelles.

Moreover, sedimentation by centrifugation separates the nanoparticles from all the hydrosoluble components.

**Table 3**

| Just prepared | | Centrifugated gel | | Filtered gel | | Centrifugated in dextran and resuspended | |
|---|---|---|---|---|---|---|---|
| Average dimension (nm) | polydispersion | Average dimension (nm) | polydispersion | Average dimension (nm) | polydispersion | Average dimension (nm) | polydispersion |
| 158.1 | 0.140 | 158.9 | 0.153 | 160.3 | 0.135 | 176.7 | 0.074 |

### EXAMPLE 4

### Loading and incorporation of dyes in the nanoparticles of trimyristin, tripalmitin and cholesteryl palmitate.

The lipid nanoparticles were loaded with two lipophilic fluorescent dyes: Nile red and 6-coumarin.

These dyes were added to the container prior to heating.

The excess dye was subsequently removed following spontaneous sedimentation.

The analysis of the incorporation took into account two parameters:
- loading capacity (i.e. the ratio between the conveyed dye and the lipid);
- incorporation efficiency (i.e. the ratio between the dye incorporated in the lipid matrix and the total dye present in the formulation).

In detail, the techniques for measuring the dye incorporation efficiency in the nanoparticles are: molecular exclusion techniques (gel filtration, gel centrifugation) and sedimentation.

In order to evaluate the incorporation efficiency, the formulations of trimyristin 1, trimyristin 2 and trimyristin 3 loaded with 6-coumarin and Nile red were analyzed.

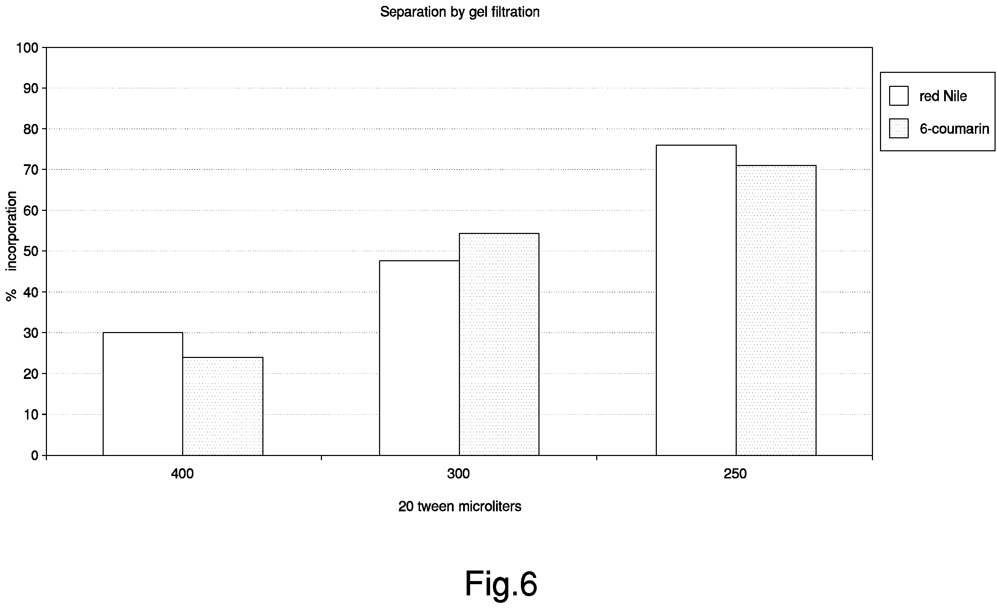

As can be seen in Figure 6, separation by gel centrifugation resulted in an incorporation efficiency greater than 90%.

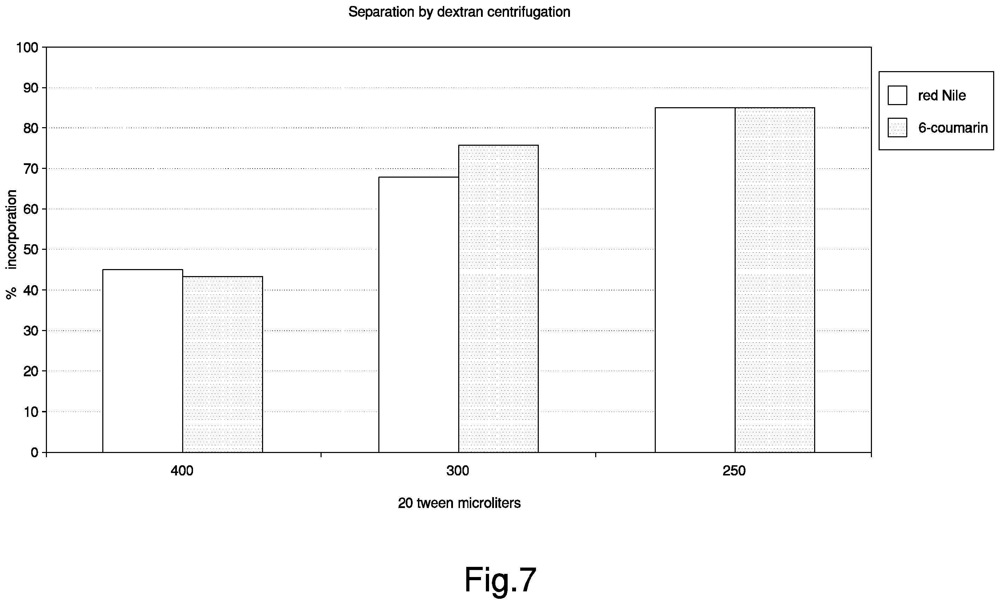

The results obtained by gel filtration and sedimentation are shown in Figures 6 and 7. In particular, the loading capacity was calculated following 30% dextran centrifugation and resuspension of the trimyristin and cholesteryl palmitate nanoparticles loaded with 6-coumarin.

Subsequently, 100 microliters of the suspended lipid nanoparticles were taken and subsequently diluted in 900 microliters of ethanol in order to extract the dye. The results obtained are shown below (Table 4).

**Table 4**

| Lipid nanoparticles | Mg dye/g lipid |
|---|---|
| Trimyristin 1 | 1.20 |
| Trimyristin 2 | 1.35 |
| Trimyristin 3 | 1.75 |
| Cholesteryl palmitate 1 | 72 |
| Cholesteryl palmitate 2 | 138 |

From the above data, it is evident that the Cholesteryl palmitate shows a surprisingly higher loading capacity than trimyristin.

### EXAMPLE 5

### Cytotoxicity of the trimyristin 2 nanoparticles.

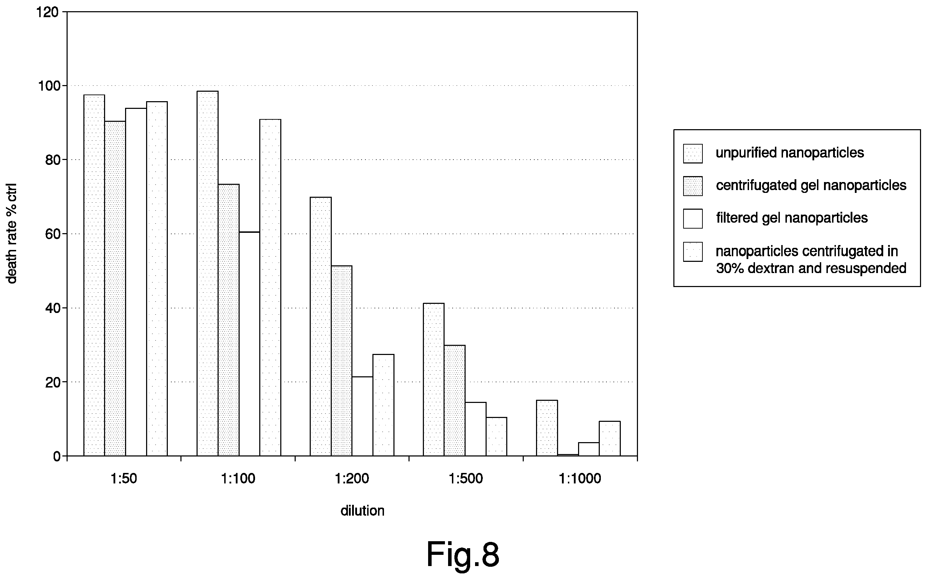

Cytotoxicity was studied on tumor cell lines with the MTT assay after 72 hours of incubation (Figure 8).

In fact, despite the excipients used for the preparation of the nanoparticles being safe and biocompatible, surfactants may cause cytotoxicity after long exposure on cells. Therefore, the effect of the surfactant purification method (Tween 20) on cytotoxicity after 72 hours of exposure was considered. Purification was performed using the methods described in example 3.

As can be seen in Figure 8, the purification technique considerably affects the cytotoxicity of lipid nanoparticles.

Gel filtration is in fact able to ensure the lowest cytotoxicity. Purification by centrifugation with 30% dextran and resuspension makes it possible to minimize cytotoxicity only after 1:200 dilution of the sample in culture medium.

This suggests that the gel filtration method is the most effective in removing the surfactant. On the contrary, gel centrifugation does not seem to work due to the molecular exclusion range used (6000 Da), which does not permit separating surfactant micelles from the nanoparticles.

### EXAMPLE 6

### Biodistribution of lipid nanoparticles marked with 6-coumarin in Wistar rats (250g) after intravenous administration.

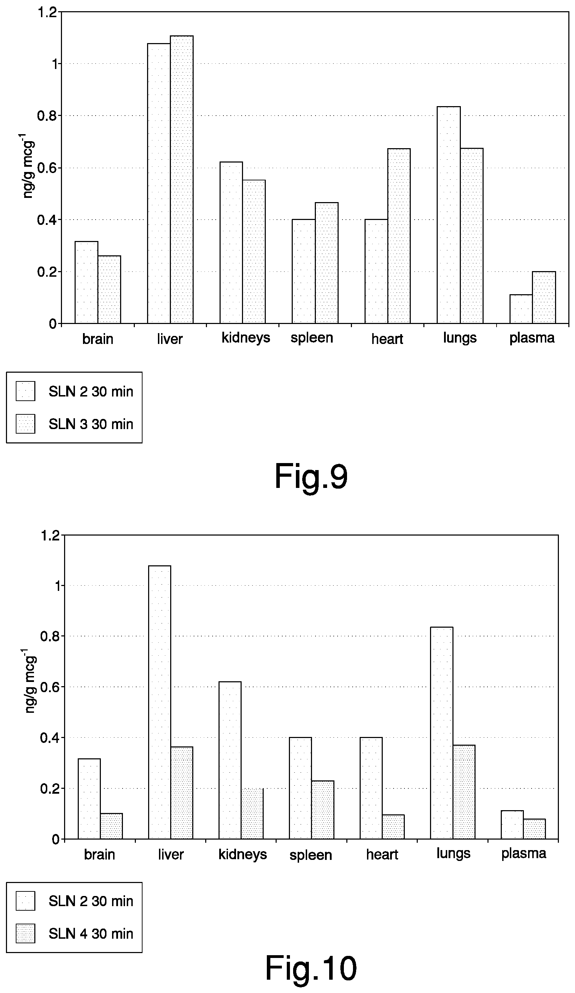

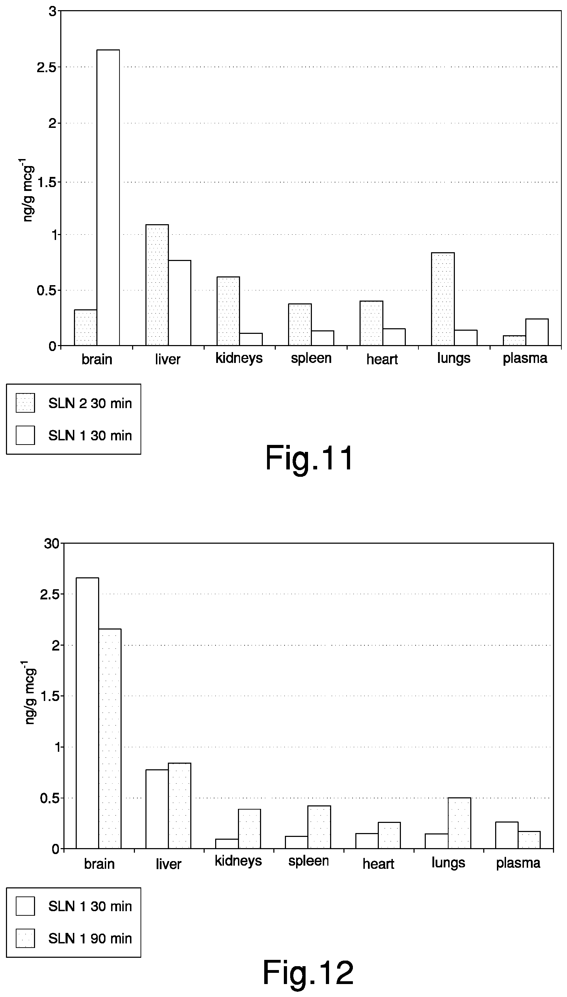

The following formulations were used (Figures 9-12):

**Table 5**

| Solid lipid nanoparticles | SLN 1 | SLN2 | SLN3 | SLN4 |
|---|---|---|---|---|
| Cholesteryl palmitate (mg) | 100 | 100 | 30 | 100 |
| Tripalmitin (mg) | | | 70 | |
| Span 80 (µl) | 200 | 200 | 200 | 200 |
| Tween 20 (µl) | 350 | 375 | 375 | |
| Tween 80 (µl) | | | | 500 |
| Water (ml) | 5 | 5 | 5 | 5 |
| Purification | Centrifugation in 30% dextran | Gel filtration (dilution 1:2) | Gel filtration (dilution 1:2) | Gel filtration (dilution 1:2) |
| Average dimension (nm) | 170.1 | 148.1 | 246.5 | 221.8 |
| Polydispersion | 0.130 | 0.124 | 0.105 | 0.227 |

The tissues were homogenized 1:4 with water and the blood centrifuged at 4000 rpm. Plasma and homogenate were diluted 1:4 with methanol and centrifuged. The supernatant was then injected into an HPLC.

There seems to be no difference in the biodistribution of nanoparticles formulated with different lipids or surfactants. There seems to be an influence of the method of purification of nanoparticles on their biodistribution: particles purified with 30% dextran show a significant increase in accumulation at brain level; this can be put down to the higher concentration of residual surfactant present in suspension.

It has in practice been ascertained that the described invention achieves the intended objects.

In particular, the fact is underlined that the particular solution of providing a mixture comprising an aqueous solution with a solid lipid matrix and at least one non-ionic biocompatible surfactant inserted in a single container, permits obtaining concentrated solid lipid nanoparticles, avoiding dilutions.

## Claims

1. Method for the preparation of lipid nanoparticles, comprising:
- the phase of mixing in a single container a mixture comprising an aqueous solution with a solid lipid matrix, in turn, comprising a matrix of lipophilic nature, insoluble in water and soluble in organic solvents, and with at least one biocompatible non-ionic surfactant and a co-surfactant which is an ester of sorbitan ;
- the phase of heating said container to a working temperature of less than 100°C to obtain an emulsion;
- the phase of cooling said emulsion up to a temperature of less than 30°C to obtain solid lipid nanoparticles having a size ranging from 40 nm to 500 nm as measured according to the description;
the phase of purification of the solid lipid nanoparticles selected from the group comprising: molecular exclusion chromatography, sedimentation and resuspension;
**characterized by** the fact that the temperature of the heating phase is higher than the lipid melting point and the cloud point of the surfactant and lower than the boiling point of the water; and by the fact that said solid lipid matrix comprises at least one of either triglyceride, a mixture of aliphatic or sterol esters.

2. Method according to one or more of the preceding claims, **characterized by** the fact that said surfactant is a polysorbate.

3. Method according to one or more of the preceding claims, **characterized by** the fact that said surfactant is a derivative of sorbitan ethoxylate.

4. Method according to one or more of the preceding claims, **characterized by** the fact that said lipid matrix is present in a weight/volume percentage concentration, evaluated with respect to the total weight of the aqueous solution, ranging from 0.3% to 5% and preferably from 0.5% to 4%.

5. Method according to one or more of the preceding claims, **characterized by** the fact that said surfactant is present in a weight/volume percentage concentration, evaluated with respect to the total weight of the aqueous solution, ranging from 1% to 20% and preferably from 2% to 16%.

6. Method according to one or more of the preceding claims, **characterized by** the fact that a co-surfactant is present in a weight/volume percentage concentration, evaluated with respect to the total weight of the aqueous solution, ranging from 0.5% to 10%, preferably from 1% to 8%.

7. Use of lipid nanoparticles obtained according to one or more of the preceding claims, to formulate radio-frequency drugs, anticancer drugs, radiopharmaceuticals.

## Patentansprüche

1. Verfahren zur Herstellung von Lipidnanopartikeln, umfassend:
- die Phase des Mischens einer Mischung in einem einzigen Behälter, die eine wässrige Lösung mit einer festen Lipidmatrix umfasst, die ihrerseits eine Matrix lipophiler Natur umfasst, die in Wasser unlöslich und in organischen Lösungsmitteln löslich ist, und mit mindestens einem biokompatiblen nichtionischen Tensid und einem Co-Tensid, das ein Sorbitanester ist;
- die Phase des Erhitzens des Behälters auf eine Arbeitstemperatur von weniger als 100 °C, um eine Emulsion zu erhalten;
- die Phase des Abkühlens der Emulsion auf eine Temperatur von weniger als 30 °C, um feste Lipidnanopartikel zu erhalten, die eine Größe im Bereich von 40 nm bis 500 nm aufweisen, gemessen gemäß der Beschreibung;
die Phase der Reinigung der festen Lipidnanopartikel, ausgewählt aus der Gruppe umfassend: Molekularausschlusschromatographie, Sedimentation und Resuspension;
**dadurch gekennzeichnet, dass** die Temperatur der Phase des Erhitzens höher ist als der Schmelzpunkt des Lipids und der Trübungspunkt des Tensids und niedriger als der Siedepunkt des Wassers; und dadurch, dass die feste Lipidmatrix mindestens eine der folgenden Komponenten umfasst: Triglycerid, eine Mischung aus aliphatischen Estern oder Sterolestern.

2. Verfahren nach einem oder mehreren der vorstehenden Ansprüche, **dadurch gekennzeichnet, dass** das Tensid ein Polysorbat ist.

3. Verfahren nach einem oder mehreren der vorstehenden Ansprüche, **dadurch gekennzeichnet, dass** das Tensid ein Derivat von Sorbitanethoxylat ist.

4. Verfahren nach einem oder mehreren der vorstehenden Ansprüche, **dadurch gekennzeichnet, dass** die Lipidmatrix in einer Gewichts-/Volumenprozentkonzentration, bezogen auf das Gesamtgewicht der wässrigen Lösung, im Bereich von 0,3 % bis 5 % und vorzugsweise von 0,5 % bis 4 % vorliegt.

5. Verfahren nach einem oder mehreren der vorstehenden Ansprüche, **dadurch gekennzeichnet, dass** das Tensid in einer Gewichts-/Volumenprozentkonzentration, bezogen auf das Gesamtgewicht der wässrigen Lösung, im Bereich von 1% bis 20 % und vorzugsweise von 2 % bis 16 % vorliegt.

6. Verfahren nach einem oder mehreren der vorstehenden Ansprüche, **dadurch gekennzeichnet, dass** ein Co-Tensid in einer Gewichts-/Volumenprozentkonzentration, bezogen auf das Gesamtgewicht der wässrigen Lösung, im Bereich von 0,5 % bis 10 %, vorzugsweise von 1 % bis 8 %, vorliegt.

7. Verwendung von Lipidnanopartikeln, die gemäß einem oder mehreren der vorstehenden Ansprüche erhalten wurden, zur Formulierung von Radiofrequenz-Arzneimitteln, Krebsmedikamenten und Radiopharmazeutika.

## Revendications

1. - Procédé de préparation de nanoparticules lipidiques, comprenant :
- la phase de mélange dans un seul récipient d'un mélange comprenant une solution aqueuse avec une matrice lipidique solide, comprenant à son tour une matrice de nature lipophile, insoluble dans l'eau et soluble dans les solvants organiques, et avec au moins un tensioactif non ionique biocompatible et un co-tensioactif qui est un ester de sorbitan ;
- la phase du chauffage dudit récipient à une température de travail de moins de 100 °C pour obtenir une émulsion ;
- la phase de refroidissement de ladite émulsion jusqu'à une température de moins de 30 °C pour obtenir des nanoparticules lipidiques solides ayant une taille se situant dans la plage de 40 nm à 500 nm, telle que mesurée conformément à la description ;
- la phase de purification des nanoparticules lipidiques solides choisie parmi le groupe comprenant : la chromatographie d'exclusion moléculaire, la sédimentation et la remise en suspension,
**caractérisé par le fait que** la température de la phase de chauffage est supérieure au point de fusion des lipides et au point de trouble de l'agent tensioactif et inférieure au point d'ébullition de l'eau ; et **par le fait que** ladite matrice lipidique solide comprend au moins l'un parmi les triglycérides et un mélange d'esters aliphatiques ou de stérols.

2. - Procédé selon l'une ou plusieurs des revendications précédentes, **caractérisé par le fait que** ledit tensioactif est un polysorbate.

3. - Procédé selon l'une ou plusieurs des revendications précédentes, **caractérisé par le fait que** ledit tensioactif est un dérivé d'éthoxylate de sorbitan.

4. - Procédé selon l'une ou plusieurs des revendications précédentes, **caractérisé par le fait que** ladite matrice lipidique est présente en une concentration en pourcentage en poids/volume, évaluée par rapport au poids total de la solution aqueuse, se situant dans la plage 0,3 % à 5 % et de préférence de 0,5 % à 4 %.

5. - Procédé selon l'une ou plusieurs des revendications précédentes, **caractérisé par le fait que** ledit tensioactif est présent en une concentration en pourcentage en poids/volume, évaluée par rapport au poids total de la solution aqueuse, se situant dans la plage de 1 % à 20 % et de préférence de 2 % à 16 %.

6. - Procédé selon l'une ou plusieurs des revendications précédentes, **caractérisé par le fait qu'**un co-tensioactif est présent en une concentration en pourcentage en poids/volume, évaluée par rapport au poids total de la solution aqueuse, se situant dans la plage de 0,5 % à 10 %, de préférence de 1 % à 8 %.

7. - Utilisation de nanoparticules lipidiques obtenues selon l'une ou plusieurs des revendications précédentes, pour formuler des médicaments associés à un traitement de radiofréquence, des médicaments anticancéreux, des produits radiopharmaceutiques.
